(19) Europäisches Patentamt
European Patent Office
Office européen des brevets

(11) EP 2 215 578 B1

(12) EUROPEAN PATENT SPECIFICATION

(45) Date of publication and mention
of the grant of the patent:
**26.03.2014 Bulletin 2014/13**

(51) Int Cl.:
*G06F 19/28* (2011.01)  *G06F 19/22* (2011.01)
*G06F 19/24* (2011.01)

(21) Application number: **07816282.3**

(22) Date of filing: **29.11.2007**

(86) International application number:
**PCT/CH2007/000599**

(87) International publication number:
**WO 2009/067823 (04.06.2009 Gazette 2009/23)**

(54) **METHOD AND COMPUTER SYSTEM FOR ASSESSING CLASSIFICATION ANNOTATIONS ASSIGNED TO DNA SEQUENCES**

VERFAHREN UND COMPUTERSYSTEM ZUR BEWERTUNG VON DNA-SEQUENZEN ZUGEWIESENEN KLASSIFIKATIONSANNOTATIONEN

PROCÉDÉ ET SYSTÈME INFORMATIQUE PERMETTANT D'ÉVALUER DES ANNOTATIONS DE CLASSIFICATION ATTRIBUÉES À DES SÉQUENCES D'ADN

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**11.08.2010 Bulletin 2010/32**

(73) Proprietor: **Smartgene GmbH**
**6300 Zug (CH)**

(72) Inventors:
• **EMLER, Stefan**
  **CH-8032 Zürich (CH)**
• **MICHEL, Pierre-André**
  **CH-1202 Genève (CH)**

(74) Representative: **Müller-Boré & Partner**
**Patentanwälte PartG mbB**
**Friedenheimer Brücke 21**
**80639 München (DE)**

(56) References cited:
**WO-A-2007/072214**

• **MA QICHENG ET AL: "Clustering protein sequences with a novel metric transformed from sequence similarity scores and sequence alignments with neural networks" BMC BIOINFORMATICS, vol. 6, October 2005 (2005-10), pages 1-13, XP002472808 ISSN: 1471-2105**
• **LEVY EMMANUEL D ET AL: "Probabilistic annotation of protein sequences based on functional classifications -" BMC BIOINFORMATICS, vol. 6, December 2005 (2005-12), pages 1-12, XP002472809 ISSN: 1471-2105**
• **KREBS WERNER G ET AL: "Statistically rigorous automated protein annotation" BIOINFORMATICS (OXFORD), vol. 20, no. 7, 1 May 2004 (2004-05-01), pages 1066-1073, XP002472810 ISSN: 1367-4803**

EP 2 215 578 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the Invention

The present invention relates to a computer-implemented method and a computer system for assessing classification annotations assigned to DNA sequences. Specifically, the present invention relates to a computer-implemented method and a computer system for assessing classification annotations assigned to DNA sequences stored in a database.

Background of the Invention

Sequence-based identification of life forms is increasingly used for diagnostic purposes. Being independent of growth and metabolism, this method offers significant advantages over conventional culture-based techniques in terms of speed and accuracy. Conserved genes present in all bacteria or fungi are amplified and subsequently sequenced using automated sequencing techniques. The sequences obtained are then compared to references in a database. Thus, even rare, unexpected or unusual isolates can be rapidly identified and classified. Sequence analysis can be applied to all conserved genes of all life-forms, particularly to microorganisms such as bacteria and fungi. Sequence-based identification of microorganisms relies on comparison of the sample signature sequence to a database containing reference sequences representing all relevant genus and species. It is therefore important that a reference database fulfills the following requirements:

> 1) Accurate sequence: the database contains correct sequences of the requested target, no sequencing errors, no reading flaws, no artificial gaps, insertions, no vector sequences.

> 2) Correct classification annotation (i.e. naming of entries): sequences are correctly annotated (e.g. species names) and this information is updated with regard to changes in taxonomy.

> 3) Representative: the database represents all relevant life-forms, e.g. genus and species, including their genetic variants (intra-species, intra-genomic).

> 4) Up-to-date: the references are up-to-date with regard to recently described species and potential changes in taxonomy (see also 2).

Currently there is no single reference database which fulfils all these requirements. However, because the quality of results of sequence comparisons greatly depends on the available references, it is crucial that these databases be as reliable as possible. In general, scientists add entries to public repositories which are of fair quality in terms of sequence content and annotation (e.g. species name). Nevertheless, there are many sequencing errors or incorrect annotations with regard to current taxonomy. Annotation errors occur, for example, when sequences are submitted along with incorrect information about the organism or gene from which the sequence has been derived, or with species names which are not up-to-date (e.g. when species have been reclassified taxonomically, as is often the case for bacteria). When a sample sequence is searched against a reference database, the resulting list usually displays indistinguishably correct and incorrect matches, leaving it up to the expertise of the user to determine references which were identified correctly or incorrectly. Thus, a correct sequence with an incorrect annotation could appear on top of the list of matches and, therefore, indicate an erroneous identification of a bacterium, for example. Because sequence-based pathogen identification is becoming nowadays part of the routine work in medical diagnostic, veterinary and industry laboratories, there is a need to render sequence database searches and comparisons easy and reliable, e.g. for identifying a bacterial or fungal species or a virus subtype, or for matching any unknown organism to a database of well characterized organisms. Particularly, the results of searching and comparing sequence similarity need to be provided adequately with regard to the expertise of routine lab technicians, who in general do not have a research background or extensive training in bio-informatics or (micro-) organism taxonomy.

US 2007/0083334 describes systems and methods for annotating biomolecular sequences. Subsequent to sequence alignment(s), biomolecular sequences are computationally clustered according to a progressive homology range using one or more clustering algorithms. A biomolecular sequence is considered to belong to a cluster, if the sequence shares an alignment-based sequence homology above a certain threshold to one member of the cluster. According to US 2007/0083334, computational clustering can be effected using any commercially available alignment software including a local homology algorithm. For example, a group exhibits a certain degree of homology, if the nucleic acids are 90% identical to one another.

US 2007/0134692 describes an alignment-based method and system for updating probe array annotation data. One or more clusters are generated by transcript across datasets retrieved from one or more sources. One or more probe sequence is aligned to a representative sequence from one or more of the clusters. The representative sequence is aligned to a genome sequence and the genome sequence is annotated with probe location information. The aligned probe sequences are mapped to the genome sequence using the alignment of the representative sequence and genome sequence. A score is computed using a number associated with the aligned probe sequences and a number associated with the probe location formation associated with a region of the genome sequence that corresponds to the aligned representative sequence. Redundant entries may be eliminated using the clustering method. For example, if the alignment of transcripts in a cluster overlap by >97% over their entire length, then they are determined to be redundant and only the longest sequence is kept in the cluster.

Summary of the Invention

It is an object of this invention to provide a computer-implemented method and a computer system for assessing (and re-assessing) classification annotations, including taxonomic, systematic and/or functional annotations, assigned to DNA sequences. In particular, it is an object of the present invention to provide a computer-implemented method and a computer system for assessing qualitatively the classification annotations such that erroneous and/or doubtful annotations become easily apparent to lab technicians who do not have extensive experience or training in bio-informatics or (micro-) organism taxonomy.

According to the present invention, these objects are achieved particularly through the features of the independent claims. In addition, further advantageous embodiments follow from the dependent claims and the description.

According to the present invention, the above-mentioned objects are particularly achieved in that, for assessing classification annotations (including taxonomic, systematic and/or functional annotations) assigned to DNA sequences stored in a database, e.g. reference database, the DNA sequences are grouped based on their respective classification annotations by species using established classification schemes for taxonomic, systematic and/or functional classification. Subsequently, for pairs of the DNA sequences, determined is in each case a measure of distance between the respective DNA sequences. The measure of distance is determined by aligning automatically the respective DNA sequences and defining the measure of distance based on a score of similarity between the aligned DNA sequences. For example, the measure of distance between two DNA sequences is calculated as a complementary value to the score of similarity, e.g. by subtracting a weighted score of similarity from one. For example, the weighted score of similarity is calculated by dividing the score of similarity between the two DNA sequences through the smaller length of the two DNA sequences. Subsequently, determined is a centroid sequence having the shortest aggregate measure of distance to the DNA sequences. Preferably, within a defined group of DNA sequences, e.g. DNA sequences related to one species, the centroid sequence is the one of these DNA sequences that has the shortest accumulated measure of distance to the other DNA sequences in the group. Alternatively, the centroid sequence is an entirely virtual object, calculated to have the lowest average measure of distance to all the DNA sequences to be considered. It should be noted that within the present context, the term "centroid sequence" is used to include a centroid object representative of an actual DNA sequence as well as a centroid object representative of a virtual object. Assigned to each one of the DNA sequences to be considered is the measure of distance between the respective one of the DNA sequences and the centroid sequence, as a quantitative confidence level for the classification annotation of the respective one of the DNA sequences. Preferably, the confidence levels are stored in the database assigned to the respective annotation and DNA sequence which match a known species or genus name. The assessment and rating of the classification annotations with these confidence levels makes it possible to provide to a user an indication of the degree of representativeness of a DNA sequence for a particular species. For example, when a user performs a query on the database, with each entry in the list of matching reference sequences a field is displayed for the user, indicating the level of confidence that the respective DNA sequence is representative for that particular species and/or genus. Depending on the embodiment, the quantitative confidence level, i.e. the measure of distance to a centroid sequence, is a numeric value or a qualitatively descriptive value derived from the numeric value. For numeric confidence levels, a small measure of distance indicates a trustworthy annotation, whereas with a greater distance, the entry should be considered more carefully with regards to providing a valid identification.

According to the present invention, the measure of distance is determined between DNA sequences within a species and centroid sequences are determined for the DNA sequences within each of the species. In a preferred embodiment, outliers are defined within the species, whereby the outliers are those DNA sequences that have the greatest measures of distance to the centroid sequence of the respective species. For example, one or more outliers are defined based on a maximum distance threshold, a defined deviation from an average measure of distance, or a defined number or quantity of DNA sequences having the largest measure of distance from the centroid sequence. For outliers which have a smaller measure of distance to a centroid sequence of another species, the annotations are marked as incorrect, e.g. by setting a respective indicator in the database.

In an embodiment, an edge-weighted graph is generated from the scores of similarity between the DNA sequences. In this graph, the DNA sequences are nodes in the graph, and the nodes are connected, if the score of similarity between the respective DNA sequences is positive (unalignable and dissimilar sequences are assigned a similarity of zero). The measure of distance between the respective DNA sequences is assigned in each case an edge weight. For the nodes in the graph, local connectivity densities (number of connections to other nodes) are computed. Clusters of nodes are defined through progressive aggregation to local connectivity density maxima, whereby the measure of distance between DNA sequences associated with nodes within a cluster (intra-cluster distance) is significantly shorter than an average measure of distance between the DNA sequences associated with the nodes of the graph (average graph distance).

In a further embodiment, a cluster threshold is received in the computer from the user, e.g. in response to the user viewing the graph shown on a display. Subsequently, the clusters of nodes are defined by applying the cluster threshold as a maximum intra-cluster distance. Thus, nodes associated with DNA sequences having a measure of distance greater than the maximum intra-cluster distance are not included in the cluster. After application of the cluster threshold, the graph is shown on the display. By selecting different cluster thresholds, the user is enabled to select a level of granularity of the graph in the sense that with a relatively high value of the cluster threshold, the graph is typically a coherent structure connecting all nodes, whereas for smaller cluster thresholds, the graph typically disintegrates into multiple clusters.

Preferably, in the graph-based approach, the DNA sequence associated with the node having the highest connectivity density in a cluster, i.e. the highest number of connections to other nodes, is defined the centroid sequence of that cluster.

In an embodiment, the classification annotation associated with a centroid sequence is assigned to DNA sequences associated with that centroid sequence. Specifically, the annotation of the centroid of a particular cluster is assigned to DNA sequences associated with the nodes of that cluster. Preferably, this annotation does not overwrite the existing classification annotation of a DNA sequence but is added as a recommendation which can be displayed to users.

In addition to a computer-implemented method and a computer system for assessing classification annotations assigned to DNA sequences stored in a database, the present invention also relates to a computer program product including computer program code means for controlling one or more processors of a computer, such that the computer performs the method, particularly, a computer program product including a computer readable medium containing therein the computer program code means.

Brief Description of the Drawings

The present invention will be explained in more detail, by way of example, with reference to the drawings in which:

Figure 1 shows a block diagram illustrating schematically an exemplary configuration of a computer-based system for practicing embodiments of the present invention, said configuration comprising a computer system with a database, and said configuration being connected to a data entry terminal via a telecommunications network.

Figure 2 shows a flow diagram illustrating an exemplary sequence of steps for rating classification annotations assigned to DNA sequences.

Figure 3 shows a flow diagram illustrating an exemplary sequence of steps for determining one or more centroid sequences.

Figure 4 shows an example of a cluster of DNA sequences related to a centroid sequence.

Figure 5 shows an alignment of 11 exemplary variations of DNA sequences related to a species.

Figure 6 shows an example of a user interface showing to a user possible matches for a sample sequence, each possible match being indicated with a confidence level (dist).

Detailed Description of the Preferred Embodiments

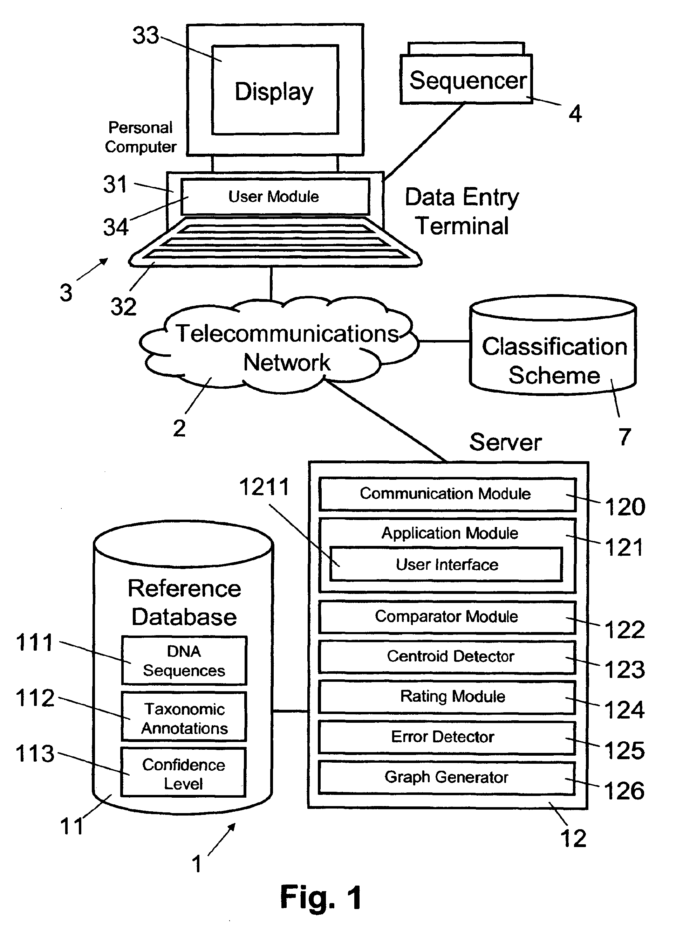

In Figure 1, reference numeral 3 refers to a data entry terminal. As illustrated in Figure 1, the data entry terminal 1 includes a personal computer 31 with a keyboard 32 and a display monitor 33, for example.

As is illustrated in Figure 1, the data entry terminal 3 is connected to computer system 1 through telecommunications network 2. Preferably, the telecommunications network 2 includes the Internet and/or an Intranet, making computer system 1 accessible as a web server through the World Wide Web or within a separate IP-network, respectively. Telecommunications network 2 may also include another fixed network, such as a local area network (LAN) or an integrated services digital network (ISDN), and/or a wireless network, such as a mobile radio network (e.g. Global System for Mobile communication (GSM) or Universal Mobile Telephone System (UMTS)), or a wireless local area network (WLAN). In a variant, at least one data entry terminal 3 is connected directly to computer system 1.

Computer system 1 includes one or more computers, each having one or more processors. Moreover, the computer system 1 comprises a (reference) database 11 including stored entries of reference DNA sequences 111. As illustrated schematically in Figure 1, computer system 1 includes different functional modules, namely a communication module 120, an application module 121, a comparator module 122, a centroid detector 123, a rating module 124, an error detector 125, and a graph generator 126. Database 11 is implemented on a computer shared with the functional modules or on a separate computer. As is illustrated schematically in Figure 1, reference database 11 includes classification annotations 112, including taxonomic, systematic and/or functional annotations, associated with DNA sequences 111. Typically, the content of reference database 11 includes entries related to DNA sequences retrieved and obtained from different (public or private) DNA sequence databases. The communication module 120 includes conventional hardware and software elements configured for exchanging data via telecommunications network 2 with one or more data entry terminals 3. The application module 121 is a programmed software module configured to provide users of the data entry terminal 3 with a user interface 1211. Preferably, user interface 1211 is provided through a conventional Internet browser such as Microsoft Explorer or Mozilla Firefox. The comparator module 122, the centroid detector 123, the rating module 124, the error detector 125, and the graph generator 126 are preferably programmed software modules executing on a processor of computer system 1.

Reference numeral 7 refers to a (networked) classification scheme database accessible to computer system 1 via telecommunications network 2. The classification scheme database includes current established classification schemes for the taxonomic, systematic and/or functional classification of DNA sequences of life forms. The classification schemes are non-static and subject to change and/or addition.

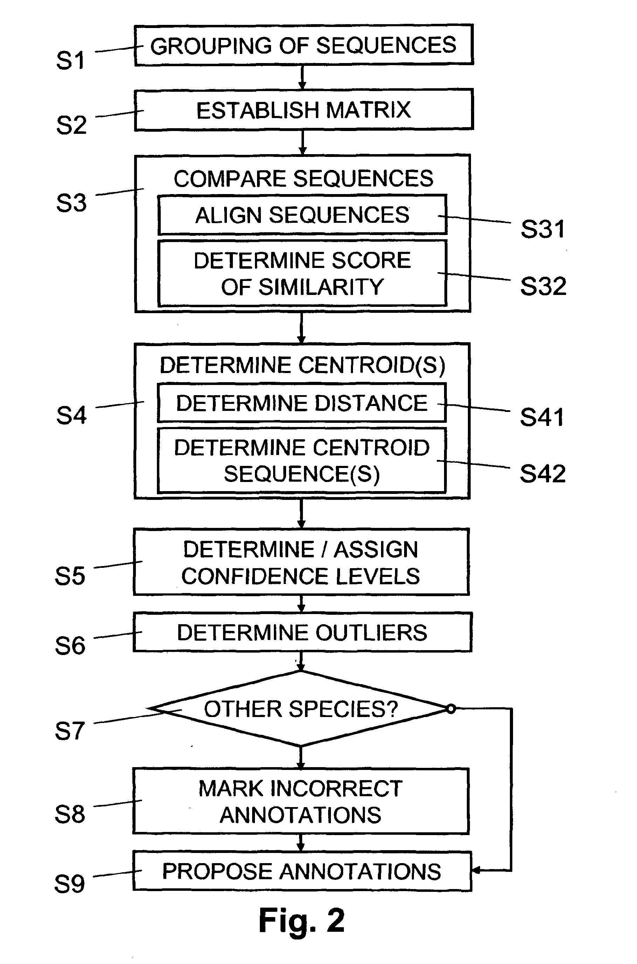

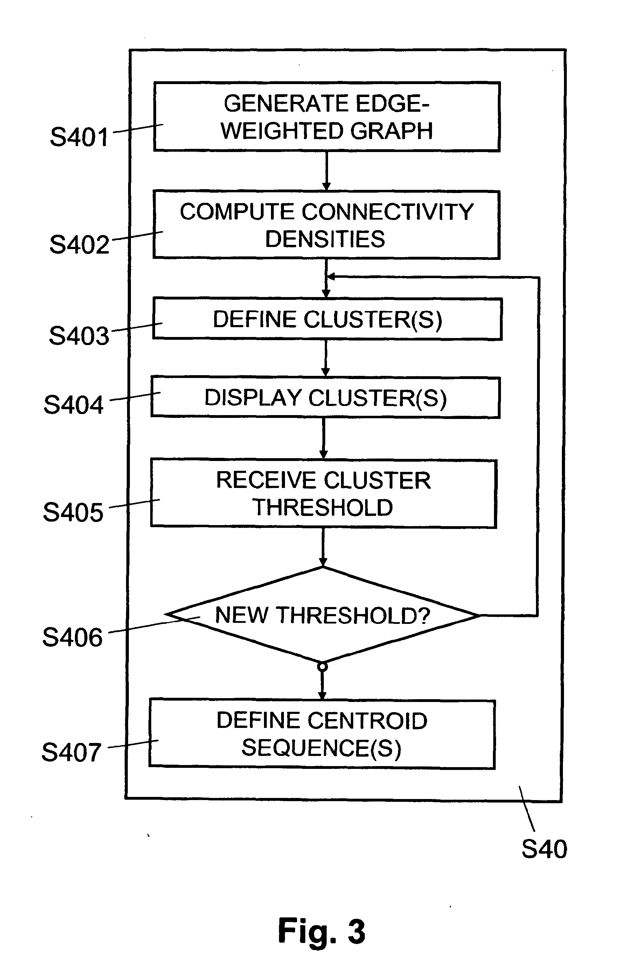

In the following paragraphs the functionality of the functional modules is described with reference to Figures 2 and 3.

In step S1, based on their respective classification annotations 112, the comparator module 122 groups by species the DNA sequences 111 stored in reference database 11 using current established classification schemes available from the classification scheme database 7. The grouping of the DNA sequences is performed for all the DNA sequences 111 or for a selected group of the DNA sequences 111. For example, the comparator module 122 is activated by an operator command a user request. In an embodiment, the comparator module 122 is activated periodically or automatically whenever a change, addition or update occurred to the classification scheme 7, or a defined number of new DNA sequences 111 have been entered (added) in the reference database 11 and/or associated with a species. Consequently, the classification annotations 112 assigned to DNA sequences 111 are assessed and re-assessed continuously and repeatedly, e.g. depending on changes in the reference database 11 and/or the classification scheme database 7.

In step S2, the comparator module 122 generates a matrix for comparing the (selected) DNA sequences 111. Depending on the embodiments, one common matrix is generated for all the DNA sequences 111, or different matrices are generated for each species.

In step S3, the comparator module 122 compares the (selected) DNA sequences 111. First the respective DNA sequences are aligned automatically in step S31.

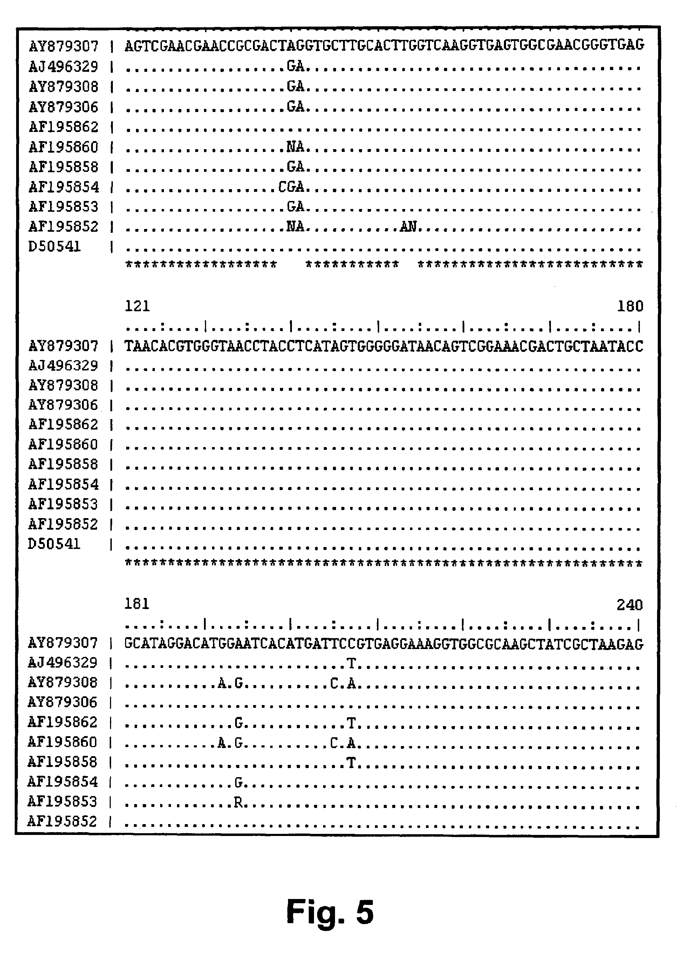

Figure 5 shows an example of an alignment of eleven sequences (e.g. bacterial ribosomal sequences, commonly used for bacterial sequence-based species identification and taxonomy) representing "Abiotrophia defectiva". As can be seen in Figure 5, these sequences are not identical; they carry differences or mutations which may either reflect sequencing errors or reflect true intraspecies or intragenomic variations. From the alignment of these sequences, it becomes apparent that these variations are often grouped and that it is possible to determine a sequence which represents best the alignment (here AY879307) and, therefore, also the bacterial species with the annotation "Abiotrophia defectiva", with regard to all published "Abiotrophia defectiva" 16S rDNA sequences that are considered.

In step S32, the comparator module 122 determines a score of similarity between the aligned DNA sequences 111, e.g. a score expressed as a percentage of sequence correspondence. The scores of similarity between the (selected) DNA sequences 111 are stored in the matrix. It must be emphasized that the score of similarity may be determined using various different alignment algorithms, e.g. pair wise, global, local, weighted and/or profile-based alignment algorithms, and taking into consideration other elements from the annotations than the classification information.

In step S4, centroid sequence(s) C are determined for the (selected) DNA sequences 111. First, in step S41, the comparator module 122 determines a measure of distance between the respective (selected) DNA sequences 111. The measure of distance is determined based on the scores of similarity between the aligned DNA sequences 111. In an embodiment, the measure of distance is determined between DNA sequences 111 within a species. Preferably, the measures of distance between the (selected) DNA sequences 111 are stored in the matrix.

For example, the measure of distance $dist(x, y)$ between two DNA sequences $x$ and $y$ is calculated by determining a complementary value of the score of similarity, e.g. $dist(x,y) = 1 - score(x,y)$. Preferably, the measure of distance $dist(x, y)$ between two DNA sequences $x$ and $y$ is calculated by determining a complementary value of a weighted score of similarity e.g. by subtracting the weighted score of similarity from one, the weighted score of similarity being calculated by dividing the score of similarity between the two aligned DNA sequences $x$, $y$ through the smaller length $l_x$, $l_y$ of the two DNA sequences $x$, $y$:

$$dist(x, y) = 1 - \frac{score(x, y)}{\min(l_x, l_y)}.$$

In step S42, based on the measures of distance, the centroid detector 123 determines the centroid sequence(s) C for the (selected) DNA sequences 111. Essentially, for each of the grouped species, the centroid sequence C is the DNA sequence in the group which has the shortest aggregate measure of distance $D$ to the other DNA sequences in the group. Alternatively, a centroid sequence $C$ is defined as a virtual object which is determined to have the shortest possible measure of distance to all the DNA sequences in the group. In other words, $c$ is the centroid sequence of a set of sequences $S$, if for all $N$ sequences $s$ in set $S$ different from $c$:

$$D(c) < D(s),$$

where

$$D(s_i) = \sum_{j=1}^{N} dist(s_i, s_j).$$

There may be more than one (congruent) centroid sequence C for DNA sequences having identical measures of distance.

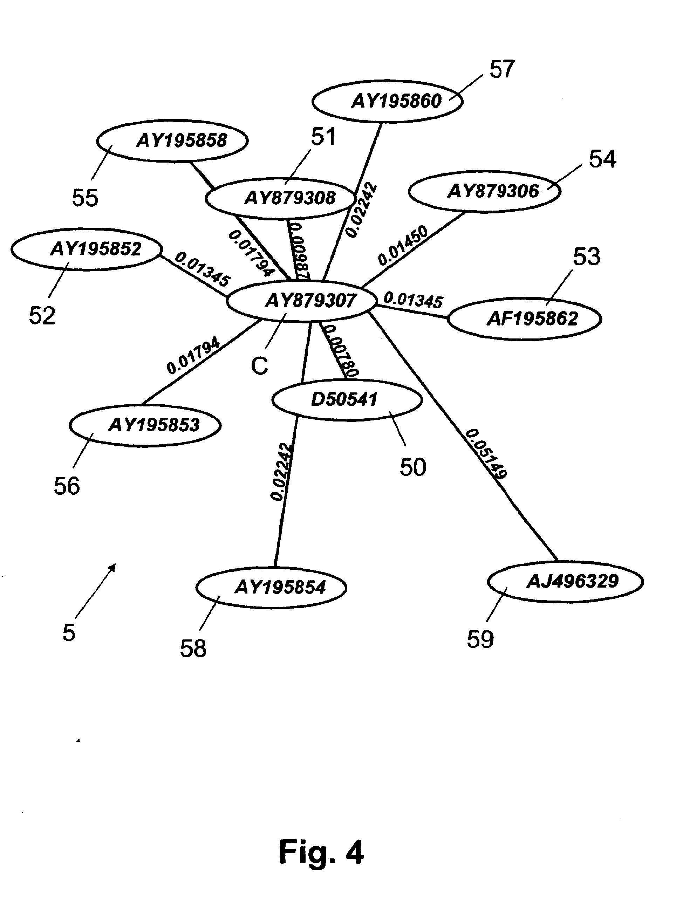

Figure 4 shows an example of ten DNA sequences 50-59, representing "Abiotrophia defectiva" as shown in Figure 5, with their respective measures of distance $dist_i(x,y)$ to the centroid sequence C ("AY879307").

In step S5, the rating module 124 assigns to the (selected) DNA sequences 111 the measure of distance $dist_i(x,y)$ between the respective DNA sequence i and the centroid sequence C as a quantitative confidence level for the classification annotation assigned to the respective DNA sequence. The smaller the measure of distance associated with a sequence, the higher the likelihood that this particular sequence is close to the centroid and thus carries its annotation correctly. Thus, a small value of the measure of distance $dist_i(x,y)$ indicates a high level of confidence; whereas a great value of the measure of distance $dist_i(x,y)$ indicates a low level of confidence. One skilled in the art will understand, that the level of confidence assigned to the (selected) DNA sequences 111 may alternatively be expressed as a complimentary quantitative value of the measure of distance $dist_i(x,y)$ or as a qualitative confidence value derived from the measure of distance $dist_i(x,y)$, e.g. from a set of verbal attributes (e.g. "very high", "high", "medium", "low", "very low") or a set of colors.

In optional step S6, the error detector 125 identifies outliers among the DNA sequences of a species. Outliers have the greatest measure of distance to the centroid sequence C of the respective species. For example, in Figure 4, DNA sequence 59 ("AJ496329") would be detected as an outlier. In an embodiment, any DNA sequence having a measure of distance to the centroid sequence C above a defined threshold or standard deviation is determined an outlier. In an embodiment, outliers are identified and removed, before determining the centroid sequences (again).

Subsequently, in step S7, the error detector 125 determines whether or not a detected outlier has a smaller measure of distance to a centroid sequence of another species. If that is the case, in step S8, the classification annotation of the outlier is marked as incorrect in reference database 11, e.g. by setting a flag field. In addition, in an embodiment, the classification annotation of the closer centroid sequence is stored assigned to the outlier as a proposed classification annotation.

In a further optional step S9, aside from outliers, the centroid detector 123 assigns the classification annotation associated with a centroid sequence C to the DNA sequences 50-58 associated with that centroid sequence C.

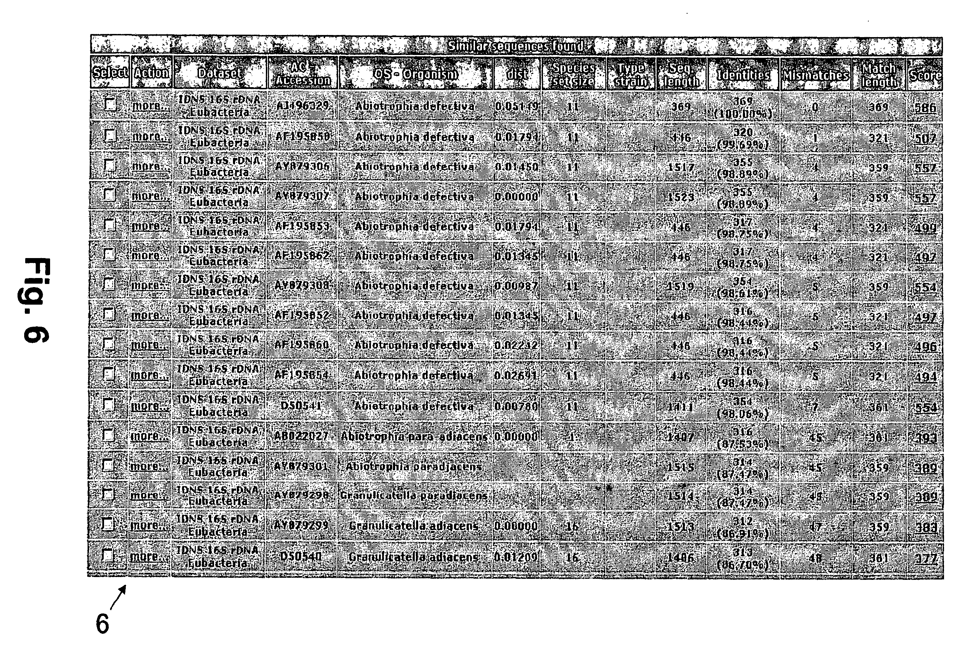

If a user accesses computer system 1 to search the reference database 11 with an uploaded DNA sequence sample, e.g. using sequence data of DNA fragments from a DNA sample from a sequencer 4 or from another source, the user is shown a user interface with a list of possible matches 6 as shown in Figure 6, for example. As can be seen in Figure 6, each list entry is provided with its respective measure of distance (dist) to the centroid C as an indicator of the level of confidence. Typically, the list is presented with a ranking by similarity and the level of confidence is used by a user as a measure of reliability of the respective classification annotation. Furthermore, outliers can be visually marked in the list, e.g. through highlighting or coloring, selectively shown or hidden from the list, and alternative classification annotations having a better confidence level can be displayed, e.g. as a proposal of a more suitable classification. The level of confidence values can further be included and displayed in any groupings, alignments, or ranked lists of DNA sequences as well as in phylogenetic trees, for example.

Figure 3 shows an exemplary sequence of steps for an extended mode of determining the centroid sequences of the (selected) DNA sequences 111. In essence, step S40 is an alternative or complementary approach to the centroid detection performed in step S4. Processing of step S40 may be triggered upon user selection or detection of a level of complexity by the centroid detector 123. The level of complexity may be indicated, for example, by at least a defined number of DNA sequences which have a measure of distance therein between exceeding a complexity threshold.

In step S401, using the scores of similarity stored in the matrix, the graph generator 126 generates an edge-weighted graph 5. The nodes in the graph are representative of the (selected) DNA sequences C, 50-59. Initially, the nodes are connected, if the score of similarity between the respective DNA sequences is positive, i.e. if it is not zero. An initial connectivity threshold may be set for the score of similarity to ensure that the nodes form one coherent graph. A measure of distance between the respective DNA sequences is assigned in each case as an edge weight between the respective nodes. The measure of distance is calculated, for example, as described above in the context of step S41.

In step S402, the graph generator 126 computes the local connectivity densities for the nodes in the graph. The local connectivity density of a node is defined by the number of connections to other nodes in the graph.

In step S403, the graph generator 126 defines clusters of nodes in the graph. The clusters are defined through progressive aggregation to local connectivity density maxima in the graph. Essentially, the measure of distance between DNA sequences associated with nodes within a cluster are significantly shorter than an average measure of distance between the DNA sequences associated with the nodes of the graph. An initial cluster threshold (allowing a large intra-cluster distance) may be defined for the measure of distance between DNA sequences associated with nodes of a cluster so that the whole graph forms just one cluster.

In step S404, the cluster is shown through user interface 1211 to a user on display 33 of data entry terminal 3.

In step S405, optionally, an alternative value for the cluster threshold is received through user interface 1211 from the user at the data entry terminal 3. If it is determined in step S406 that a new cluster threshold was received from the user, the graph generator 126 defines the clusters in step S403 using the new cluster threshold as a maximum intra-cluster distance. Subsequently, the graph with the newly defined cluster is displayed in step S404. If it is determined in step S406 that no new cluster threshold was received from the user, processing continues in step S407.

In step S407, the centroid detector 123 determines the centroid sequence(s) C for the one or more clusters of the graph. For each cluster, the centroid detector 123 determines the DNA sequence associated with the node having the highest connectivity density in the cluster as the centroid sequence C of that cluster. Subsequently processing continues in step S5 as described above with reference to Figure 2.

It should be noted that, in the description, the computer program code has been associated with specific functional modules and the sequence of the steps has been presented in a specific order, one skilled in the art will understand, however, that the computer program code may be structured differently and that the order of at least some of the steps could be altered, without deviating from the scope of the invention. It should also be noted that the proposed method and system cannot only be used for off-line assessment of classification annotations in a database, but also online (real-time or near real-time), e.g. as a filter for entering the classification annotation for a new DNA sequence to be added to a database.

**Claims**

1. A computer-implemented method of assessing classification annotations (112) assigned to DNA sequences (111) stored in a database (11), the method comprising:

   grouping (S1) the DNA sequences (111) based on their respective classification annotations (112) by species using established classification schemes;
   determining for pairs of the DNA sequences (111) a measure of distance between the respective DNA sequences (111) by aligning (S31) automatically the respective DNA sequences (111) and determining the measure of distance (S41) based on a score of similarity between the aligned DNA sequences (111);
   determining a centroid sequence (S4, S40), the centroid sequence (C) having a shortest aggregate measure of distance to the DNA sequences (111); and
   assigning (S5) to the DNA sequences (111) the measure of distance between the respective DNA sequence and the centroid sequence (C) as a quantitative confidence level for the classification annotation assigned to the respective DNA sequence;
   wherein the measure of distance is determined between DNA sequences (111) within a species; and centroid sequences are determined for the DNA sequences (111) within each of the species.

2. The method according to claim 1, further comprising identifying outliers within the species, the outliers having a greatest measure of distance to the centroid sequence (C) of the respective species, and marking annotations (112) as incorrect for outliers which have a smaller measure of distance to a centroid sequence (C) of another species.

3. The method according to one of claims 1 or 2, wherein the method further comprises generating (S401) from the scores of similarity between the DNA sequences (111) an edge-weighted graph, the DNA sequences (111) being nodes in the graph, the nodes being connected, if the score of similarity between the respective DNA sequences (111) is positive, and the measure of distance between the respective DNA sequences (111) being assigned in each case as an edge weight; computing (S402) local connectivity densities for the nodes in the graph; and defining clusters (S403) of nodes through progressive aggregation to local connectivity density maxima, the measure of distance between DNA sequences (111) associated with nodes within a cluster being significantly shorter than an average measure of distance between the DNA sequences (111) associated with the nodes of the graph, wherein preferably the method further comprises receiving a cluster threshold (S405) from a user, responsive to showing the graph on a display (33); defining the clusters (S403) of nodes by applying the cluster threshold as a maximum intra-cluster distance; and showing the graph (S404) on the display (33) after applying the cluster threshold.

4. The method according to claim 3, wherein the DNA sequence associated with the node having the highest connectivity density in a cluster is defined the centroid sequence (C) of that cluster.

5. The method according to one of claims 1 to 4, wherein the classification annotation associated with a centroid sequence (C) is assigned to DNA sequences (111) associated with that centroid sequence (C).

6. The method according to one of claims 1 to 5, wherein determining the measure of distance (S41) between two DNA sequences (111) includes calculating a weighted score of similarity by dividing the score of similarity between the two DNA sequences (111) through the smaller length of the two DNA sequences (111), and subtracting the weighted score of similarity from one.

7. A computer system (1) for assessing classification annotations (112) assigned to DNA sequences (111), the system (1) comprising:

a database (11) comprising a plurality of the DNA sequences (111);
a comparator module (122) configured to group the DNA sequences (111) based on their respective classification annotations (112) by species using established classification schemes (7), and to determine for pairs of the DNA sequences (111) a measure of distance between the respective DNA sequences (111) by aligning automatically the respective DNA sequences (111) and determining the measure of distance based on a score of similarity between the aligned DNA sequences (111);
a centroid detector (123) configured to determine a centroid sequence (C), the centroid sequence (C) having a shortest aggregate measure of distance to the DNA sequences (111); and
a rating module (124) configured to assign to the DNA sequences (111) the measure of distance between the respective DNA sequence and the centroid sequence (C) as a quantitative confidence level for the classification annotation assigned to the respective DNA sequence;
wherein the comparator module (122) is further configured to determine the measure of distance between DNA sequences (111) within a species; and the centroid detector (123) is further configured to determine the centroid sequences (C) for the DNA sequences (111) within each of the species.

8. The system (1) according to claim 7, further comprising an error detector (125) configured to identify outliers within the species, the outliers having a greatest measure of distance to the centroid sequence (C) of the respective species, and to mark annotations (112) as incorrect for outliers which have a smaller measure of distance to a centroid sequence (C) of another species.

9. The system (1) according to one of claims 7 or 8, wherein the system (1) further comprises a graph generator (126) configured to generate from the scores of similarity between the DNA sequences (111) an edge-weighted graph, the DNA sequences (111) being nodes in the graph, the nodes being connected, if the score of similarity between the respective DNA sequences (111) is positive, and the measure of distance between the respective DNA sequences (111) being assigned in each case as an edge weight, to compute local connectivity densities for the nodes in the graph, and to define clusters of nodes through progressive aggregation to local connectivity density maxima, the measure of distance between DNA sequences (111) associated with nodes within a cluster being significantly shorter than an average measure of distance between the DNA sequences (111) associated with the nodes of the graph.

10. The system (1) according to claim 9, wherein the system (1) further comprises a user interface (1211) configured to receive a cluster threshold from a user, responsive to showing the graph on a display (33); the graph generator (126) is further configured to define the clusters of nodes by applying the cluster threshold as an maximum intra-cluster distance, and to show the graph on the display (33) after applying the cluster threshold.

11. The system (1) according to one of claims 9 or 10, wherein the centroid detector (123) is further configured to define the DNA sequence associated with the node having the highest connectivity density in a cluster as the centroid sequence (C) of that cluster.

12. The system (1) according to one of claims 7 to 11, wherein the centroid detector (123) is further configured to assign the classification annotation associated with a centroid sequence (C) to DNA sequences (111) associated with that centroid sequence (C).

13. The system (1) according to one of claims 7 to 12, wherein the comparator module (122) is further configured to determine the measure of distance between two DNA sequences (111) by subtracting a weighted score of similarity from one, the weighted score of similarity being calculated by dividing the score of similarity between the two DNA sequences (111) through the smaller length of the two DNA sequences (111).

14. A computer program product comprising computer program code means for controlling one or more processors of a computer system (1), such that the computer system (1) performs the method according to one of the claims 1 to 6.

15. The computer program product according to claim 14, further comprising a computer readable medium containing therein the computer program code means.

**Patentansprüche**

1. Computer-implementiertes Verfahren zum Beurteilen von Klassifizierungsnotierungen (112), welche in einer Datenbank (11) gespeicherten DNA-Sequenzen (111) zugeordnet sind, wobei das Verfahren umfaßt:

Gruppieren (S1) der DNA-Sequenzen (111) nach ihrer Spezies, basierend auf ihren jeweiligen Klassifizierungsnotierungen (112), unter Verwendung etablier-ter Klassifizierungsschemata,

Bestimmen eines Maßes der Distanz zwischen den jeweiligen DNA-Sequenzen (111) für Paare von DNA-Sequenzen (111) durch automatisches Ausrichten (*aligning*; S31) der jeweiligen DNA-Sequenzen (111) und Bestimmen des Maßes der Distanz (S41), basierend auf einem Ähnlichkeitswert zwischen den ausgerichteten DNA-Sequenzen (111),

Bestimmen einer Centroid-Sequenz (S4, S40), wobei die Centroid-Sequenz (C) ein kürzestes Gesamtmaß der Distanz zu den DNA-Sequenzen (111) aufweist, und

Zuordnen (S5) des Maßes der Distanz zwischen der jeweiligen DNA-Sequenz und der Centroid-Sequenz (C) als ein quantitatives Konfidenzniveau für die Klassifizierungsnotierung, welche der jeweiligen DNA-Sequenz zugeordnet ist, zu den DNA-Sequenzen (111),

wobei das Maß der Distanz zwischen DNA-Sequenzen (111) innerhalb einer Spezies bestimmt wird und Centroid-Sequenzen für die DNA-Sequenzen (111) innerhalb jeder der Spezies bestimmt werden.

2. Verfahren nach Anspruch 1, weiter umfassend das Identifizieren von Ausrei-ßern innerhalb der Spezies, wobei die Ausreißer ein größtes Maß der Distanz zu der Centroid-Sequenz (C) der jeweiligen Spezies aufweisen und das Markieren von Notierungen (112) als nicht korrekt für Ausreißer, welche ein kleineres Maß der Distanz zu einer Centroid-Sequenz (C) einer anderen Spezies aufweisen.

3. Verfahren nach einem der Ansprüche 1 oder 2, wobei das Verfahren weiter das Erzeugen (S401) eines kantengewichteten Diagramms aus den Ähnlichkeitswerten zwischen den DNA-Sequenzen (111), wobei die DNA-Sequenzen (111) Knotenpunkte in dem Diagramm darstellen, wobei die Knotenpunkte verbunden sind, wenn der Ähnlichkeitswert zwischen den jeweiligen DNA-Sequenzen (111) positiv ist und das Maß der Distanz zwischen den jeweiligen DNA-Sequenzen (111) in jedem Fall als eine Kantengewichtung zugeordnet wird; das Berechnen (S402) lokaler Konnektivitätsdichten für die Knotenpunkte in dem Diagramm; und das Definieren von Anhäufungen (S403) von Knotenpunkten durch progressive Aggregation zu lokalen Konnektivitätsdichtenmaxima, wobei das Maß der Distanz zwischen den DNA-Sequenzen (111), die mit den Knotenpunkten innerhalb einer Anhäufung assoziiert sind, signifikant kürzer ist als ein Durchschnittsmaß der Distanz zwischen den DNA-Sequenzen (111), die mit den Knotenpunkten des Diagramms assoziiert sind, umfaßt, wobei das Verfahren vorzugsweise weiter das Erhalten eines Anhäufungsschwellenwerts (S405) von einem Benutzer, in Antwort auf das Darstellen des Diagramms auf einem Display (33); das Definieren der Anhäufungen (S403) von Knotenpunkten durch Anwenden des Anhäufungsschwellenwerts als eine maximale Distanz innerhalb der Anhäufungen; und das Darstellen des Diagramms (S404) auf dem Display (33), nachdem der Anhäufungsschwellenwert angewendet wurde, umfaßt.

4. Verfahren nach Anspruch 3, wobei die DNA-Sequenz, die mit dem Knotenpunkt assoziiert ist, welcher die höchste Konnektivitätsdichte innerhalb einer Anhäufung aufweist, als die Centroid-Sequenz (C) dieser Anhäufung definiert wird.

5. Verfahren nach einem der Ansprüche 1 bis 4, wobei die Klassifizierungsnotierung, die mit einer Centroid-Sequenz (C) assoziiert ist, DNA-Sequenzen (111) zugeordnet wird, die mit dieser Centroid-Sequenz (C) assoziiert sind.

6. Verfahren nach einem der Ansprüche 1 bis 5, wobei das Bestimmen des Ma-ßes der Distanz (S41) zwischen zwei DNA-Sequenzen (111) das Berechnen eines gewichteten Ähnlichkeitswerts durch Teilen des Ähnlichkeitswerts zwischen den beiden DNA-Sequenzen (111) durch die kleinere Länge der beiden DNA-Sequenzen (111) und das Subtrahieren des gewichteten Ähnlichkeitswerts von eins umfaßt.

7. Computersystem (1) für das Beurteilen von Klassifizierungsnotierungen (112), die DNA-Sequenzen (111) zugeordnet sind, wobei das System (1) umfaßt:

Eine Datenbank (11), umfassend eine Vielzahl von DNA-Sequenzen (111),
ein Vergleichsmodul (122), das ausgelegt ist, die DNA-Sequenzen (111) nach Spezies, basierend auf ihren jeweiligen Klassifizierungsnotierungen (112), unter Verwendung von etablierten Klassifizierungsschemata (7) zu gruppieren, und für Paare der DNA-Sequenzen (111) ein Maß der Distanz zwischen den jeweiligen DNA-Sequenzen (111) durch automatisches Ausrichten der jeweiligen DNA-Sequenzen (111) und das Maß der Distanz, basierend auf einem Ähnlichkeitswert zwischen den ausgerichteten DNA-Sequenzen (111) zu bestimmen,
einen Centroid-Detektor (123), der ausgelegt ist, eine Centroid-Sequenz (C) zu bestimmen, wobei die Centroid-Sequenz (C) ein kürzestes Gesamtmaß der Distanz zu den DNA-Sequenzen (111) aufweist, und
ein Bewertungsmodul (124), das ausgelegt ist, den DNA-Sequenzen (111) das Maß der Distanz zwischen der jeweiligen DNA-Sequenz und der Centroid-Sequenz (C) als ein quantitatives Konfidenzniveau für die Klassifkationsnotierung, welcher der jeweiligen DNA-Sequenz zugeordnet ist, zuzuordnen,
wobei das Vergleichsmodul (122) weiter ausgelegt ist, das Maß der Distanz zwischen DNA-Sequenzen (111) innerhalb einer Spezies zu bestimmen, und der Centroid-Detektor (123) weiter ausgelegt ist, die Centroid-Sequenzen (C) für die DNA-Sequenzen (111) innerhalb jeder der Spezies zu bestimmen.

8. System (1) nach Anspruch 7, weiter umfassend einen Fehlerdetektor (125), der ausgelegt ist, Ausreißer innerhalb der Spezies zu identifizieren, wobei die Ausreißer ein größtes Maß der Distanz zu der Centroid-Sequenz (C) der jeweiligen Spezies aufweisen, und Notierungen (112) für Ausreißer, welche ein kleineres Maß der Distanz zu einer Centroid-Sequenz (C) einer anderen Spezies aufweisen, als nicht korrekt zu markieren.

9. System (1) nach einem der Ansprüche 7 oder 8, wobei das System (1) weiter einen Diagrammgenerator (126) umfaßt, der ausgelegt ist, aus den Ähnlichkeitswerten zwischen den DNA-Sequenzen (111) ein kantengewichtetes Diagramm zu erzeugen, wobei die DNA-Sequenzen (111) Knotenpunkte in dem Diagramm darstellen, wobei die Knotenpunkte verbunden sind, wenn der Ähnlichkeitswert zwischen den jeweiligen DNA-Sequenzen (111) positiv ist und das Maß der Distanz zwischen den jeweiligen DNA-Sequenzen (111) in jedem Fall als eine Kantengewichtung zugeordnet wird, um lokale Konnektivitätsdichten für die Knotenpunkte in dem Diagramm zu berechnen, und Anhäufungen von Knotenpunkten durch progressive Aggregation zu lokalen Konnektivitätsdichtenmaxima zu definieren, wobei das Maß der Distanz zwischen DNA-Sequenzen (111), die mit den Knotenpunkten innerhalb einer Anhäufung assoziiert sind, signifikant kürzer ist als ein Durchschnittsmaß der Distanz zwischen den DNA-Sequenzen (111), die mit den Knotenpunkten des Diagramms assoziiert sind.

10. System (1) nach Anspruch 9, wobei das System (1) weiter ein Benutzerinterface (1211) umfaßt, das ausgelegt ist, einen Anhäufungsschwellenwert in Antwort auf das Darstellen des Diagramms auf einem Display (33) von einem Benutzer zu erhalten, wobei der Diagrammgenerator (126) weiter ausgelegt ist, die Anhäufungen von Knotenpunkten durch Anwenden des Anhäufungsschwellenwerts als eine maximale Distanz innerhalb einer Anhäufung zu definieren, und das Diagramm auf dem Display (33) anzuzeigen, nachdem der Anhäufungsschwellenwert angewendet wurde.

11. System (1) nach einem der Ansprüche 9 oder 10, wobei der Centroid-Detektor (123) weiter ausgelegt ist, die DNA-Sequenz, die mit dem Knotenpunkt assoziiert ist, welcher die höchste Konnektivitätsdichte innerhalb einer Anhäufung aufweist, als die Centroid-Sequenz (C) dieser Anhäufung zu definieren.

12. System (1) nach einem der Ansprüche 7 bis 11, wobei der Centroid-Detektor (123) weiter ausgelegt ist, die Klassifizierungsnotierung, die mit einer Centroid-Sequenz (C) assoziiert ist, DNA-Sequenzen (111) zuzuordnen, welche mit dieser Centroid-Sequenz (C) assoziiert sind.

13. System (1) nach einem der Ansprüche 7 bis 12, wobei das Vergleichsmodul (122) weiter ausgelegt ist, das Maß der Distanz zwischen zwei DNA-Sequenzen (111) durch Subtrahieren eines gewichteten Ähnlichkeitswerts von eins zu bestimmen, wobei der gewichtete Ähnlichkeitswert durch Teilen des Ähnlichkeitswerts zwischen den beiden DNA-Sequenzen (111) durch die kleinere Länge der beiden DNA-Sequenzen (111) berechnet wird.

14. Computerprogrammprodukt, umfassend Computerprogramm-kodierte Mittel für das Kontrollieren eines oder mehrerer Prozessoren eines Computersystems (1), so daß das Computersystem (1) das Verfahren nach einem der Ansprüche 1 bis 6 ausführt.

15. Computerprogrammprodukt nach Anspruch 14, weiter umfassend ein Computer-lesbares Medium, enthaltend darauf die Computerprogramm-kodierten Mittel.

**Revendications**

1. Procédé informatique d'estimation d'annotations de classification (112) attribuées à des séquences d'ADN (111) stockées dans une banque de données (11), le procédé comprenant :

le groupement (S1) des séquences d'ADN (111) sur base de leurs annotations de classification (112) respectives par espèce en utilisant des schémas de classification établis ;
la détermination pour des paires des séquences d'ADN (111), d'une mesure de la distance entre les séquences d'ADN (111) respectives par alignement (S31) automatique des séquences d'ADN (111) respectives et détermination de la mesure de distance (S41) sur base d'un score de similarité entre les séquences d'ADN (111) alignées ;
la détermination d'une séquence centroïde (S4, S40), la séquence centroïde (C) ayant une mesure d'agrégat la plus courte de distance des séquences d'ADN (111), et
l'attribution (S5) aux séquences d'ADN (111), de la mesure de distance entre la séquence d'ADN respective et la séquence centroïde (C) comme degré de confiance quantitatif de l'annotation de classification attribuée à la séquence d'ADN respective ;
où la mesure de distance est déterminée entre les séquences d'ADN (111) dans une espèce, et les séquences centroïdes sont déterminées pour les séquences d'ADN (111) au sein de chaque espèce.

2. Procédé selon la revendication 1, comprenant en outre, l'identification de valeurs atypiques dans les espèces, les valeurs atypiques ayant la plus grande mesure de distance à la séquence centroïde (C) des espèces respectives, et le marquage d'annotations (112) en tant qu'incorrectes pour les valeurs atypiques qui ont une valeur de distance plus faible à la séquence centroïde (C) d'autres espèces.

3. Procédé selon l'une des revendications 1 et 2, où le procédé comprend en outre, la génération (S401) à partir des scores de similarité entre les séquences d'ADN (111), d'un graphique à arête pondérée, les séquences d'ADN (111) étant les noeuds dans le graphique, les noeuds étant connectés, si le score de similarité entre les séquences d'ADN (111) respectives est positif, et la mesure de distance entre les séquences d'ADN (111) respectives étant attribuée dans chaque cas comme une pondération d'arête ; le calcul (S402) des densités de connectivité locale pour les noeuds dans le graphique ; et la définition de groupements (S403) de noeuds par agrégation progressive aux maxima de densité de connectivité locale, la mesure de distance entre les séquences d'ADN (111) associées aux noeuds dans un groupement étant significativement plus faible qu'une mesure moyenne de distance entre les séquences d'ADN (111) associées aux noeuds dans le graphique, où de préférence, le procédé comprend en outre, la réception d'un seuil de groupement (S405) depuis un utilisateur, en réponse à l'apparition du graphique sur un écran (33) ; la définition des groupements (S403) de noeuds par application du seuil de groupement comme distance intra-groupement maximale ; et la représentation du graphique (S404) sur l'écran (33) après application du seuil de groupement.

4. Procédé selon la revendication 3, où la séquence d'ADN associée au noeud ayant la densité de connectivité la plus élevée dans un groupement est définie comme séquence centroïde (C) de ce groupement.

5. Procédé selon l'une quelconque des revendications 1 à 4, où l'annotation de classification associée à une séquence centroïde (C) est attribuée aux séquences d'ADN (111) associées à cette séquence centroïde (C).

6. Procédé selon l'une quelconque des revendications 1 à 5, où la détermination de la mesure de distance (S41) entre deux séquences d'ADN (111) comprend le calcul d'un score pondéré de similarité en divisant le score de similarité entre les deux séquences d'ADN (111) par la plus petite longueur des deux séquences d'ADN (111), et la soustraction du score pondéré de similarité de un.

7. Système informatique (1) pour évaluer les annotations de classification (112) attribuées à des séquences d'ADN (111), le système (1) comprenant :

une banque de données (11) comprenant une série de séquences d'ADN (111) ;
un module comparateur (122) configuré pour grouper les séquences d'ADN (111) sur base de leurs annotations de classification (112) respectives par espèce en utilisant des schémas de classification établis (7), et pour déterminer pour les paires de séquences d'ADN (111), une mesure de distance entre les séquences d'ADN (111) respectives par alignement automatique des séquences d'ADN (111) respectives et détermination de la mesure de distance sur base d'un score de similarité entre les séquences d'ADN (111) alignées ;

un détecteur de centroïde (123) configuré pour déterminer une séquence centroïde (C), la séquence centroïde (C) ayant la mesure d'agrégat la plus courte de distance aux séquences d'ADN (111), et

un module de notation (124) configuré pour attribuer aux séquences d'ADN (111), la mesure de distance entre la séquence d'ADN respective et la séquence centroïde (C) comme degré de confiance quantitatif de l'annotation de classification attribuée à la séquence d'ADN respective ;

où le module comparateur (122) est encore configuré pour déterminer la mesure de distance entre les séquences d'ADN (111) au sein d'une espèce, et le détecteur de centroïde (123) est encore configuré pour déterminer les séquences centroïdes (C) pour les séquences d'ADN (111) au sein de chaque espèce.

8. Système (1) selon la revendication 7, comprenant en outre, un détecteur d'erreur (125) configuré pour identifier les valeurs atypiques au sein des espèces, les valeurs atypiques ayant la plus grande mesure de distance à la séquence centroïde (C) des espèces respectives, et pour marquer d'annotations (112) en tant qu'incorrectes pour les valeurs atypiques qui ont une valeur de distance plus faible à la séquence centroïde (C) d'autres espèces.

9. Système (1) selon l'une des revendications 7 et 8, où le système (1) comprend en outre, un générateur de graphique (126), configuré pour générer à partir des scores de similarité entre les séquences d'ADN (111), un graphique à arête pondérée, les séquences d'ADN (111) étant les noeuds dans le graphique, les noeuds étant connectés, si le score de similarité entre les séquences d'ADN (111) respectives est positif, et la mesure de distance entre les séquences d'ADN (111) respectives étant attribuée dans chaque cas comme une pondération d'arête, pour calculer les densités de connectivité locale pour les noeuds dans le graphique, et pour définir des groupements (S403) de noeuds par agrégation progressive aux maxima de densité de connectivité locale, la mesure de distance entre les séquences d'ADN (111) associées aux noeuds dans un groupement étant significativement plus faible qu'une mesure moyenne de distance entre les séquences d'ADN (111) associées aux noeuds dans le graphique.

10. Système (1) selon la revendication 9, où le système (1) comprend en outre, une interface utilisateur (1211) configurée pour réceptionner un seuil de groupement depuis un utilisateur, en réponse à l'apparition d'un graphique sur un écran (33) ; le générateur de graphique (126) étant configuré en outre, pour définir les groupements de noeuds par application du seuil de groupement comme distance intragroupement maximale, et pour représenter le graphique sur l'écran (33) après application du seuil de groupement.

11. Système (1) selon l'une des revendications 9 et 10, où le détecteur de centroïde (123) est en outre configuré pour définir la séquence d'ADN associée au noeud ayant la densité de connectivité la plus élevée dans un groupement comme séquence centroïde (C) de ce groupement.

12. Système (1) selon l'une des revendications 7 à 11, où le détecteur de centroïde (123) est en outre configuré pour attribuer l'annotation de classification associée à une séquence centroïde (C) aux séquences d'ADN (111) associées à cette séquence centroïde (C).

13. Système (1) selon l'une des revendications 7 à 12, où le module comparateur (122) est en outre configuré pour déterminer la mesure de distance entre deux séquences d'ADN (111) par soustraction d'un score pondéré de similarité de un, le score pondéré de similarité étant calculé en divisant le score de similarité entre les deux séquences d'ADN (111) par la plus petite longueur des deux séquences d'ADN (111).

14. Produit de programme d'ordinateur comprenant un moyen de code de programme d'ordinateur pour contrôler un ou plusieurs processeurs d'un système d'ordinateur (1), de sorte que le système d'ordinateur (1) effectue le procédé selon l'une des revendications 1 à 6.

15. Produit de programme d'ordinateur selon la revendication 14, comprenant en outre, un support lisible par ordinateur contenant le moyen de code du programme d'ordinateur.

**Fig. 1**

**Fig. 2**

S401 — GENERATE EDGE-WEIGHTED GRAPH

S402 — COMPUTE CONNECTIVITY DENSITIES

S403 — DEFINE CLUSTER(S)

S404 — DISPLAY CLUSTER(S)

S405 — RECEIVE CLUSTER THRESHOLD

S406 — NEW THRESHOLD?

S407 — DEFINE CENTROID SEQUENCE(S)

S40

**Fig. 3**

**Fig. 4**

```
AY879307 | AGTCGAACGAACCGCGACTAGGTGCTTGCACTTGGTCAAGGTGAGTGGCGAACGGGTGAG
AJ496329 | ....................GA......................................
AY879308 | ....................GA......................................
AY879306 | ....................GA......................................
AF195862 | ...........................................................
AF195860 | ....................NA......................................
AF195858 | ....................GA......................................
AF195854 | ...................CGA......................................
AF195853 | ....................GA......................................
AF195852 | ....................NA..........AN..........................
D50541   | ...........................................................
           ********************  **********  ***************************

           121                                                      180
           ...:....|....:....|....:....|....:....|....:....|....:....|
AY879307 | TAACACGTGGGTAACCTACCTCATAGTGGGGGATAACAGTCGGAAACGACTGCTAATACC
AJ496329 | ...........................................................
AY879308 | ...........................................................
AY879306 | ...........................................................
AF195862 | ...........................................................
AF195860 | ...........................................................
AF195858 | ...........................................................
AF195854 | ...........................................................
AF195853 | ...........................................................
AF195852 | ...........................................................
D50541   | ...........................................................
           ***********************************************************

           181                                                      240
           ...:....|....:....|....:....|....:....|....:....|....:....|
AY879307 | GCATAGGACATGGAATCACATGATTCCGTGAGGAAAGGTGGCGCAAGCTATCGCTAAGAG
AJ496329 | ............................T..............................
AY879308 | ..........A.G..........C.A.................................
AY879306 | ...........................................................
AF195862 | ..............G...........T................................
AF195860 | ..........A.G..........C.A.................................
AF195858 | ..........................T................................
AF195854 | ..............G............................................
AF195853 | ..............R............................................
AF195852 | ...........................................................
```

# Fig. 5

17

**Fig. 6**

Similar sequences found

| Select | Action | Dataset | AC Accession | OS - Organism | dist | Species strand | Type strain | Seq. length | Identities | Mismatches | Match length | Score |
|---|---|---|---|---|---|---|---|---|---|---|---|---|
| ☐ | more... | rDNS 16S rDNA Eubacteria | AJ196329 | Abiotrophia defectiva | 0.051919 | 11 | | 369 | 369 (100.00%) | 0 | 369 | 586 |
| ☐ | more... | rDNS 16S rDNA Eubacteria | AF195958 | Abiotrophia defectiva | 0.010179 | 11 | | 320 | 355 (99.89%) | 1 | 321 | 507 |
| ☐ | more... | rDNS 16S rDNA Eubacteria | AY879307 | Abiotrophia defectiva | 0.014450 | 11 | | 1523 | 355 (98.89%) | 1 | 359 | 557 |
| ☐ | more... | rDNS 16S rDNA Eubacteria | AY879306 | Abiotrophia defectiva | 0.000000 | 11 | | 1517 | 347 (98.75%) | 4 | 321 | 499 |
| ☐ | more... | rDNS 16S rDNA Eubacteria | AF195853 | Abiotrophia defectiva | 0.011794 | 11 | | 146 | 347 (98.75%) | 4 | 321 | 497 |
| ☐ | more... | rDNS 16S rDNA Eubacteria | AF195862 | Abiotrophia defectiva | 0.013345 | 11 | | 146 | 354 (98.61%) | 5 | 359 | 554 |
| ☐ | more... | rDNS 16S rDNA Eubacteria | AY879308 | Abiotrophia defectiva | 0.009187 | 11 | | 1519 | 354 (98.61%) | 5 | 359 | 554 |
| ☐ | more... | rDNS 16S rDNA Eubacteria | AF195852 | Abiotrophia defectiva | 0.013445 | 11 | | 146 | 316 (98.44%) | 5 | 321 | 497 |
| ☐ | more... | rDNS 16S rDNA Eubacteria | AF195960 | Abiotrophia defectiva | 0.022232 | 11 | | 146 | 316 (98.44%) | 5 | 321 | 496 |
| ☐ | more... | rDNS 16S rDNA Eubacteria | AF195954 | Abiotrophia defectiva | 0.028391 | 11 | | 146 | 364 (98.37%) | 6 | 321 | 494 |
| ☐ | more... | rDNS 16S rDNA Eubacteria | AB022027 | Abiotrophia para-adiacens | 0.000000 | 1 | | 1407 | 316 (97.53%) | 7 | 361 | 293 |
| ☐ | more... | rDNS 16S rDNA Eubacteria | D50541 | Abiotrophia paradiacens | 0.008760 | 11 | | 1515 | 314 (87.78%) | 45 | 359 | 389 |
| ☐ | more... | rDNS 16S rDNA Eubacteria | AY879301 | Abiotrophia para-adiacens | 0.009750 | 16 | | 1514 | 316 (87.77%) | 45 | 361 | 389 |
| ☐ | more... | rDNS 16S rDNA Eubacteria | AY879298 | Granulicatella para-adiacens | 0.008000 | 16 | | 1515 | 314 (87.70%) | 45 | 359 | 389 |
| ☐ | more... | rDNS 16S rDNA Eubacteria | AY879299 | Granulicatella adiacens | 0.012109 | 16 | | 1515 | 312 (86.91%) | 47 | 359 | 383 |
| ☐ | more... | rDNS 16S rDNA Eubacteria | D50540 | Granulicatella adiacens | 0.012109 | 16 | | 1406 | 313 (86.70%) | 48 | 361 | 373 |

6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070083334 A
- US 20070134692 A